# EUROPEAN PATENT SPECIFICATION

(11) **EP 3 735 999 B1**
(45) Date of publication and mention of the grant of the patent: **24.07.2024**
(21) Application number: 20173582.6
(22) Date of filing: 07.05.2020
(51) Int. Cl.: A61M 5/142, A61J 15/00, F04B 43/12, F04B 49/06, A61M 39/08, A61M 39/24

(54) **ENTERAL FEEDING PUMP AND TUBING SET**
PUMPE UND SCHLAUCHSET FÜR DIE ENTERALE ERNÄHRUNG
POMPE D'ALIMENTATION ENTÉRALE ET ENSEMBLE DE TUBAGE

(30) Priority: 07.05.2019 US 201916404842
(43) Date of publication of application: 11.11.2020
(73) Proprietor: iMed Technology, Inc., Dallas, TX 75287 (US)
(72) Inventor: ADAMS, Kyle S., Dallas, Texas 75287 (US)
(74) Representative: Moore, Michael Richard

(56) References cited:
- EP-A1- 2 397 695
- US-A1- 2005 267 418
- US-B1- 6 494 692
- US-B1- 6 531 708

## Description

### FIELD OF THE INVENTION

The present application relates to generally to administration feeding sets for delivering fluids to a patient and, more particularly, to an enteral feeding pump and tubing set that facilitates secure loading of the tubing set in the enteral feeding pump

### BACKGROUND OF THE INVENTION

An enteral feeding system can be used to provide, for example, nutrient solutions to patients who may not be capable of feeding themselves. An enteral feeding system may typically include a flow control apparatus, such as an enteral feeding pump, e.g., a peristaltic pump, which can be attached to a tubing set, such as an administration feeding set, including an input tube connected to a supply container and to an output tube connected to a patient. The pump draws nutrient solution from the supply container and delivers the solution to the patient. A peristaltic pump typically comprises a housing that includes a rotor driven by a motor, such as through a gearbox connecting the motor to the rotor to drive the rotor in rotation. A section of the tubing set can be engaged around the rotor, wherein rotation of the rotor drives solution through the tubing of the tubing set by peristaltic action in which the tubing is progressively compressed to drive the solution at a controlled rate through the tubing set. A controller can operate the motor to drive the rotor at a selected rate as may be determined by an operator to provide a selected solution delivery rate, such as may be determined by flow parameters programmed into the controller.

In order for the pump to deliver an accurate amount of solution corresponding to the flow parameters programmed into the pump, the tubing set must be correctly loaded in the pump. If the tubing set is incorrectly installed in the pump, the pump may fail to operate correctly and/or fail to deliver the correct amount of solution through the tubing set. Hence, there is a continuing need for an enteral feeding pump and tubing set that is designed to facilitate proper installation of the tubing set in the pump and maintain correct operation of the pump with the tubing set. Example installations of tubing sets into infusion pumps are known from documents: US 2005/267418 A1, EP 2 397 695 A1, US 6 531 708 B1 and US 6 494 692 B1.

### SUMMARY OF THE INVENTION

In accordance with one aspect of the invention, an enteral feeding pump is provided for mounting a tubing set. The enteral feeding pump comprises a base having an outer side. A rotor is supported on the outer side of the base for engaging a tube section of a tubing set. A tubing set platform is provided on the outer side of the base and defines a pair of tube retainer locks. At least one tube retainer lock comprises an elongated channel for receiving a tube retainer defined by a retainer body and a pair of laterally opposing retainer tabs, tab rest surfaces located on laterally opposing sides of the channel, and tab retention surfaces spaced from the tab rest surfaces and defining elongated slots extending in a longitudinal direction parallel to the elongated channel. The elongated slots each include an entrance end for receiving a retainer tab and a longitudinally opposing inner end.

The elongated channel can extend below a plane defined by the tab rest surfaces.

The elongated channel can include a first portion having a first lateral width and a second portion having a second lateral width that is less than the first lateral width, the first portion extending from at least a longitudinal location aligned with the entrance ends of the slots to a longitudinal location aligned with the inner ends of the slots.

An interface surface may be provided generally perpendicular to the longitudinal direction, and may be defined at an interface between the first and second portions for engaging an end of a retainer body.

The elongated channel may further include a third portion defining a tube passage having a third lateral width that is less than the second lateral width.

The elongated slots may comprise passages for slidably receiving respective retainer tabs.

Each of the passages may comprise a continuous, straight line passage extending from the entrance end to the inner end of the slot.

At least one of the inner ends of the slots may define a stop surface for engaging and limiting sliding movement of the retainer tab within the slot.

In accordance with another aspect of the invention, an apparatus is provided comprising an enteral feeding pump and tubing set. The apparatus comprises a base having an outer side, and a rotor is supported on the outer side of the base for engaging a tube section of a tubing set comprising first and second tube retainers connected to opposing ends of the tube section. Each tube retainer comprises a retainer body and a pair of laterally opposing retainer tabs. A tubing set platform is provided on the outer side of the base and defines a pair of tube retainer locks. Each tube retainer lock comprises an elongated channel for receiving a retainer body, tab rest surfaces located on laterally opposing sides of the channel, and tab retention surfaces spaced from the tab rest surfaces defining elongated slots extending in a longitudinal direction parallel to the elongated channel, wherein the elongated slots each include an entrance end for receiving a retainer tab and a longitudinally opposing inner end.

The elongated channel may extend below a plane defined by the tab rest surfaces.

The elongated slots may comprise passages for slidably receiving respective retainer tabs.

Each of the passages may comprise a continuous, straight line passage extending from the entrance end to the inner end of the slot.

Each retainer tab includes a planar upper surface and a planar lower surface parallel to the planar upper surface.

At least one of the inner ends of the slots may define a stop surface for engaging and limiting sliding movement of the retainer tab within the slot.

Each retainer body may be a cylindrical body defining a fluid passage therethrough, and each channel may be defined by a generally semicircular surface for engaging a respective retainer body.

An inlet tube may be attached to one of the tube retainers and an outlet tube may be attached to the other of the tube retainers, and an anti-free flow check valve may be located at a distal end of the outlet tube.

In accordance with a further aspect of the invention, an apparatus is provided comprising a tubing set for an enteral feeding pump having a rotor and a pair of tube retainer locks defining channels and a pair of opposing elongated retainer slots on either side of each channel. The tubing set comprises a tube segment having opposing ends and a tube retainer attached to each of the tube ends. Each tube retainer comprises a retainer body defining a longitudinal axis and a pair of retainer tabs extending laterally outward from the longitudinal axis in opposing directions. Each retainer tab has upper and lower surfaces for guiding the tab through a respective retainer slot.

The upper and lower surfaces may comprise a planar upper surface and a planar lower surface parallel to the planar upper surface.

An inlet tube may be attached to one of the tube retainers and an outlet tube may be attached to the other of the tube retainers, and an anti-free flow check valve may be located at a distal end of the outlet tube.

In another aspect, there is provided a tubing set for an enteral feeding pump, wherein the tubing set may be defined according to any one or more of the features and embodiments of the tubing set of the second and/or third aspects of the invention; and where enteral feeding pump combinable with the tubing set of this aspect may have any one or more of the features of the enteral feeding pump of the first and/or second aspects of the invention.

In yet another aspect there is provided an enteral feeding pump according to any one or more aspect or embodiment of the invention, in combination with a tubing set according to any one or more aspect or embodiment of the invention.

It should be appreciated that any of the features and embodiments of the feeding pump of the first aspect of the invention, are intended to be combinable (unless incompatible) with any of the features and embodiments of the feeding pump of the second aspect of the invention. Moreover, any of the features and embodiments of the tubing set of the second aspect of the invention, are intended to be combinable (unless incompatible) with any of the features and embodiments of the tubing set of the third aspect of the invention. Similarly, it should be appreciated that any of the features and embodiments of the tubing set of the third aspect of the invention, are intended to be combinable (unless incompatible) with any of the features and embodiments of the tubing set of the second aspect of the invention; and any of the of the features and embodiments of the feeding pump of the second aspect of the invention, are intended to be combinable (unless incompatible) with any of the features and embodiments of the feeding pump of the first aspect of the invention. Furthermore, the feeding pump of the first aspect of the invention is intended to be compatible (although not necessarily exclusively so) with the tubing set of the third aspect of the invention and *vice versa.*

### BRIEF DESCRIPTION OF THE DRAWINGS

The appended claims define the present invention which will be better understood from the following description in conjunction with the accompanying Drawing Figures, in which like reference numerals identify like elements, and wherein:
Fig. 1 is a perspective view of an enteral feeding pump;
Fig. 2 is a diagrammatic view of a tubing set for use with the enteral feeding pump of Fig. 1;
Fig. 3 is an enlarged perspective view of a tubing set platform including tube retainer locks for receiving the tubing set shown in Fig. 2;
Fig. 4 is an enlarged perspective view of an entrance end of the tube retainer locks and cooperating tube retainers shown without tubes;
Fig. 5 is a plan view of a tubing set partially mounted in the tubing set platform;
Fig. 6 is an inlet end view of a tube retainer for attachment to a fluid supply tube of the tubing set; and
Fig. 7 is an outlet end view of a tube retainer for attachment to an outlet tube of the tubing set.

### DETAILED DESCRIPTION OF THE INVENTION

In the following detailed description of the preferred embodiment, reference is made to the accompanying drawings that form a part hereof, and in which is shown by way of illustration, and not by way of limitation, a specific preferred embodiment in which the invention may be practiced.

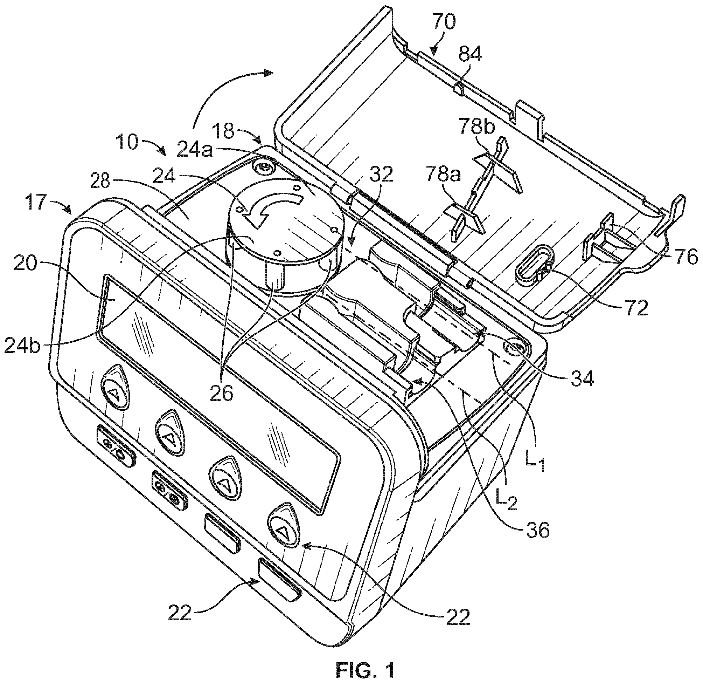

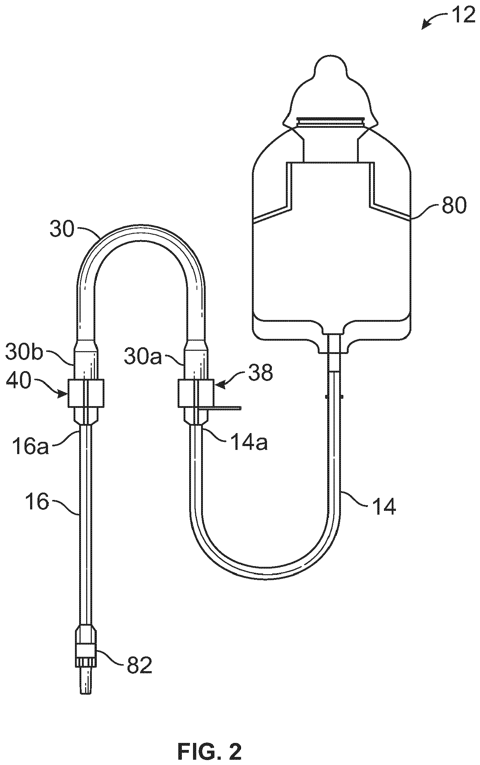

The present description is directed to an enteral feeding system including an enteral feeding pump or pump 10, see Fig. 1, and an enteral feeding set or tubing set 12, see Fig. 2, for mounting in the pump 10, wherein the pump 10 and tubing set 12 are formed with features to facilitate installation and secure placement of the tubing set 12 in the pump 10. The pump 10 operates on a tube section 30 of the tubing set 12 through peristaltic action to convey a fluid from an inlet or fluid supply tube 14 of the tubing set 12 to an outlet tube 16 of the tubing set 12.

Referring to Fig. 1, the pump 10 comprises a housing 17 including a base 18 for the pump 10. The housing 17 can include a display screen 20 on a front of the housing 17, and buttons 22 that can be used to control operation of the pump 10 via a controller (not shown) including providing control of information displayed on the display screen 20, such as information related to the pumping operation of the pump 10.

The pump 10 further includes a disk-shaped rotor 24 mounted to an outer side 28 of the base 18, wherein the rotor 24 can comprise a plurality of tube engaging rollers 26. In the illustrated embodiment, four tube engaging rollers 26 can be provided. A motor (not shown) can be located within the housing 17 for driving the rotor 24 in rotating movement to apply a peristaltic action to the tube section 30 of the tubing set 12, see Fig. 2, that can extend around the rotor 24 when the tubing set 12 is mounted in the pump 10. For example, rotation of the rotor 24 can cause the rollers 26 to successively engage with the tube section 30 to pump fluid through the tubing set 12.

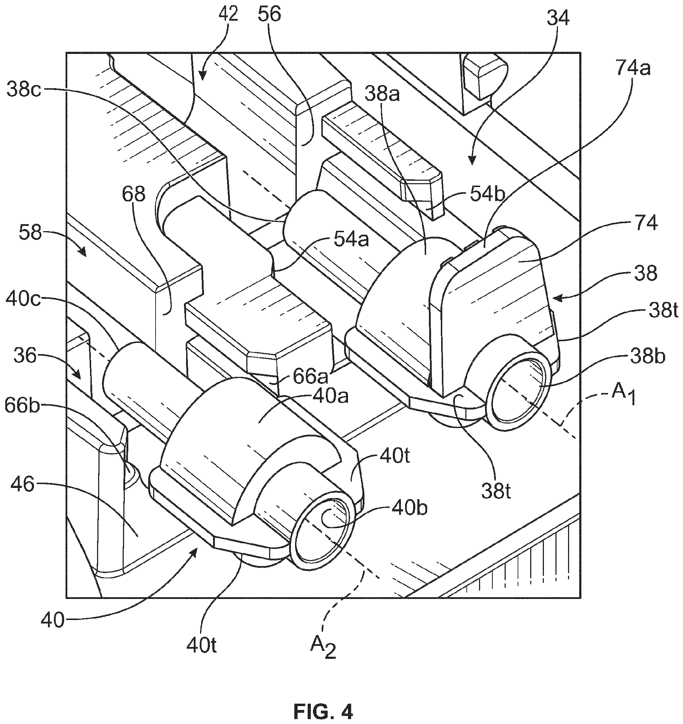

As seen in Fig. 1, a tubing set platform 32 is located on the outer side 28 of the base 18, and may be formed integrally with the base 18. The tubing set platform 32 defines first and second tube retainer locks 34, 36 located in side-by-side relation. The first tube retainer lock 34 is configured to receive a fluid supply tube retainer 38, see Fig. 4, and the second tube retainer lock 36 is configured to receive an outlet tube retainer 40, wherein the fluid supply tube retainer 38 and the outlet tube retainer 40 are provided for connecting the tube section 30 to the respective fluid supply and outlet tubes 14, 16, see Fig. 2.

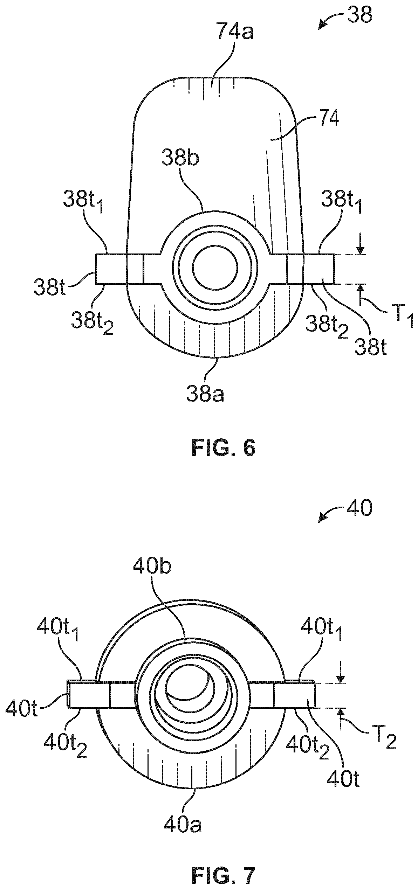

Referring to Figs. 4 and 6, the fluid supply tube retainer 38 includes a cylindrical body 38a, a first port 38b and a second port 38c, wherein the first and second ports 38b, 38c may be generally longitudinally aligned along an axis A₁ of the cylindrical body 38a. The first port 38b is configured to receive an end 14a of the fluid supply tube 14, i.e., the end 14a may be positioned within the first port 38b. The second port 38c is configured to receive a first end 30a of the tube section 30, i.e., the first end 30a may be positioned over the second port 38c. A through passage is defined generally extending along the axis A₁ through the first port 38b, the cylindrical body 38a, and the second port 38c for passage of fluid from the end 14a of the fluid supply tube 14 to the first end 30a of the tube section 30.

A pair of tabs 38t extend laterally, or radially relative to the axis A₁, from opposing sides of the cylindrical body 38a, and can further include portions extending along the first port 38b. The tabs 38t comprise thin planar structures, each tab 38t defining a generally planar upper surface 38t₁, and a generally planar lower surface 38t₂ parallel to the upper surface 38t₁. It should be noted that, as an alternative to the longitudinally continuous structure of the embodiment illustrated herein, the tabs 38t could be formed as discontinuous structures, such as may be defined by a plurality of longitudinally spaced features extending laterally from at least the cylindrical body 38a. Hence, the planar surfaces 38t₁, 38t₂ may be generally defined by a plurality of longitudinally spaced points provided on one or more features extending radially outward from either side of the cylindrical body 38a and extending parallel to the axis A₁.

Referring to Figs. 4 and 7, the outlet tube retainer 40 includes a cylindrical body 40a, a first port 40b and a second port 40c, wherein the first and second ports 40b, 40c may be generally longitudinally aligned along an axis A₂ of the cylindrical body 40a. The first port 40b is configured to receive an end 16a of the outlet tube 16, i.e., the end 16a may be positioned within the first port 40b. The second port 40c is configured to receive a second end 30b of the tube section 30, i.e., the second end 30b may be positioned over the second port 40c. A through passage is defined generally extending along the axis A₂ through the first port 40b, the cylindrical body 40a, and the second port 40c for passage of fluid from the second end 30b of the tube section 30 to the end 16a of the outlet tube 16.

A pair of tabs 40t extend laterally, or radially relative to the axis A₁, from opposing sides of the cylindrical body 40a, and can further include portions extending from the first port 40b. The tabs 40t comprise thin planar structures, each tab 40t defining a generally planar upper surface 40t₁, and a generally planar lower surface 40t₂ parallel to the upper surface 40t₁. It should be noted that, as an alternative to the longitudinally continuous structure of the embodiment illustrated herein, the tabs 40t could be formed as discontinuous structures, such as may be defined by a plurality of longitudinally spaced features extending laterally from at least the cylindrical body 40a. Hence, the planar surfaces 40t₁, 40t₂ may be generally defined by a plurality of longitudinally spaced points provided on one or more features extending radially outward from either side of the cylindrical body 40a and extending parallel to the axis A₂.

It should be understood that the present description of the tabs 38t, 40t as being "thin planar structures" refers to a thickness, e.g., a thickness dimension T₁, T₂ perpendicular relative to the lateral direction of extension of the respective tabs 38t, 40t, that is less than a diameter of the respective cylindrical body 38a, 40a. More particularly, and without limitation, the thickness T₁, T₂ of the tabs 38t, 40t can be less than 50% of the diameter of the respective cylindrical body 38a, 40a, and may be 25% or less than the diameter of the respective cylindrical body 38a, 40a.

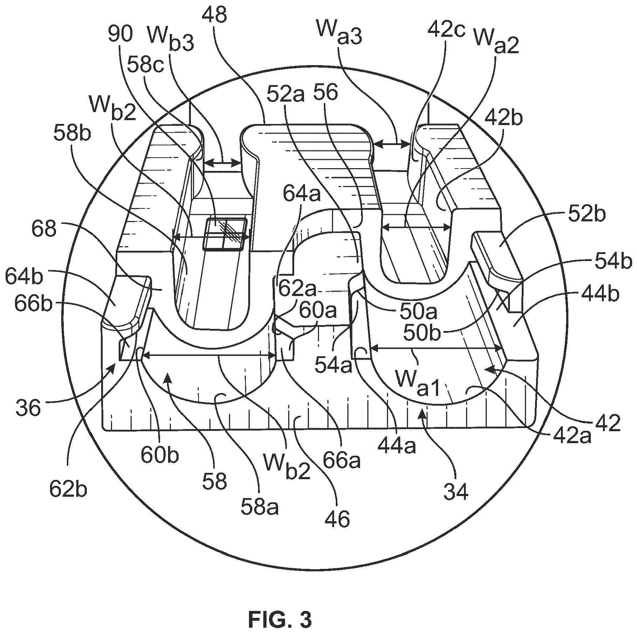

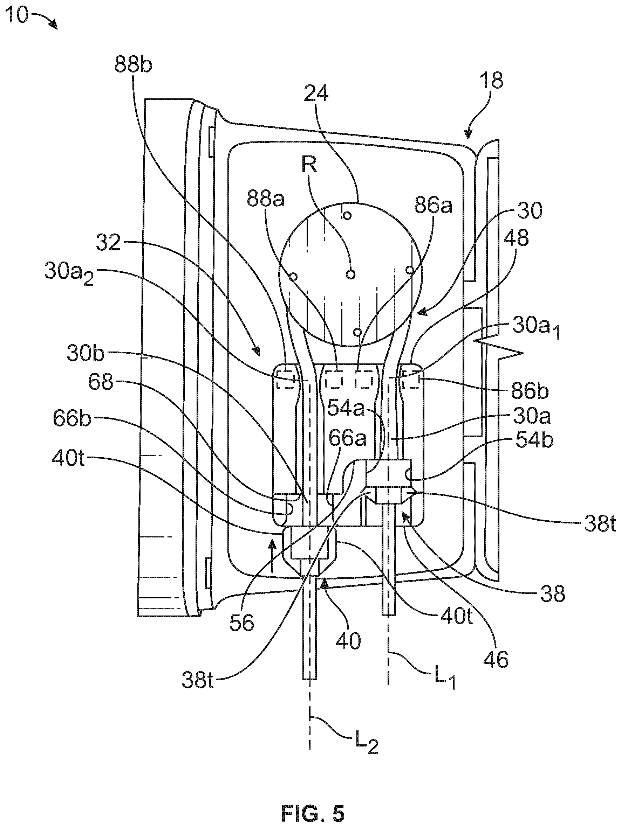

Referring to Figs. 3 and 4, the first tube retainer lock 34 comprises a first elongated recess or channel 42 defining an inlet longitudinal axis L₁, see Fig. 1, that is generally tangentially aligned with an inlet side 24a of the rotor 24 and which can be offset toward a rotational axis R, see Fig. 5, of the rotor 24 to facilitate positioning the tube section 30 for engagement with the rotor 24. The elongated channel 42 can include a first portion 42a having a first lateral width Wₐ₁, a second portion 42b having a second lateral width Wₐ₂, and a third portion 42c having a third lateral width Wₐ₃. The second lateral width Wₐ₂ is less than the first lateral width Wₐ₁, and the third lateral width Wₐ₃ is less than the second lateral width Wₐ₂, as will be discussed in greater detail below.

The first tube retainer lock 34 further includes tab rest surfaces 44a, 44b facing away from the outer side 28 of the base 18. The tab rest surfaces 44a, 44b are located on laterally opposing sides of the first portion 42a of the channel 42 and comprise elongated generally planar surfaces extending parallel the longitudinal axis L₁ in a direction from a first side 46 of the platform 32 toward a second side 48 of the platform 32, wherein the first portion 42a of the channel 42 extends below a plane defined by the tab rest surfaces 44a, 44b. Tab retention surfaces 50a, 50b defined on first retainer lock shoulder portions 52a, 52b of the platform 32 are spaced from the tab rest surfaces 44a, 44b and define elongated slots 54a, 54b extending in the longitudinal direction parallel to the elongated channel 42, i.e., parallel to the longitudinal axis L₁. Hence, each slot 54a, 54b defines a continuous, straight line passage extending from an entrance end to an inner end of the slot 54a, 54b.

A first interface surface 56 extends generally perpendicular to the longitudinal axis L₁, wherein the interface surface 56 is defined at an interface between the first and second portions 42a, 42b of the channel 42, and the slots 54a, 54b can extend along the first channel portion 42a up to the longitudinal location of the interface surface 56. In addition, at least a portion of the tab rest surfaces 44a, 44b extending from the first side 46 of the platform 32 can be provided as open support surfaces, i.e., without the shoulder portions 52a, 52b defining the slots 54a, 54b, to define shelf portions of the tab rest surfaces 44a, 44b.

The first portion 42a of the channel 42 may be formed as a generally semicircular surface, or as a section of a circle, having opposing edges at the tab rest surfaces 44a, 44b. The first portion 42a is located for engaging the retainer body 38a of the fluid supply tube retainer 38, and the tabs 38t extend radially from the retainer body 38a to laterally overlap the tab rest surfaces 44a, 44b and the tab retention surfaces 50a, 50b. Further, a vertical dimension of the slots 54a, 54b, as defined by a spacing between the tab rest surfaces 44a, 44b and the tab retention surfaces 50a, 50b can be slightly greater than the thickness T₁ of the tabs 38t to permit the tabs 38t to slide in non-binding guided movement through the slots 54a, 54b. It may be understood that a clearance can be provided between the tabs 38t and the slot surfaces to avoid frictional binding and permit free sliding movement of the tabs 38t through the slots 54a, 54b.

The second and third portions 42b, 42c of the elongated channel 42 can be formed as U-shaped surface. The width dimension Wₐ₂ of the second portion 42b may define a width sufficient to receive the first end 30a of the tube section 30 positioned over the second port 38c of the fluid supply tube retainer 38. The width dimension Wₐ₃ of the third portion 42c may define a width sufficient to receive a portion of the tube section 30 adjacent to the first end 30a of the tube section 30 positioned over the second port 38c of the fluid supply tube retainer 38, and can be dimensioned to engage opposing sides of the tube section 30, i.e., the width dimension Wₐ₃ can be the same as, or slightly greater than, the outer diameter of the tube section 30.

Also as seen in Figs. 3 and 4, the second tube retainer lock 36 comprises a second elongated recess or channel 58 defining an outlet longitudinal axis L₂, see Fig. 1, that is generally tangentially aligned with an outlet side 24b of the rotor 24 and which can be offset toward the rotational axis R, see Fig. 5, of the rotor 24 to facilitate positioning the tube section 30 for engagement with the rotor 24. The elongated channel 58 can include a first portion 58a having a first lateral width W_{b1}, a second portion 58b having a second lateral width W_{b2}, and a third portion 58c having a third lateral width W_{b3}. The second lateral width W_{b2} is less than the first lateral width W_{b1}, and the third lateral width W_{b3} is less than the second lateral width W_{b2}, as will be discussed in greater detail below.

The second tube retainer lock 36 further includes tab rest surfaces 60a, 60b facing away from the outer side 28 of the base 18. The tab rest surfaces 60a, 60b are located on laterally opposing sides of the first portion 58a of the channel 58 and comprise elongated generally planar surfaces extending parallel the longitudinal axis L₁ in a direction from the first side 46 of the platform 32 toward a second side 48 of the platform 32, wherein the first portion 58a of the channel 58 extends below a plane defined by the tab rest surfaces 60a, 60b. Tab retention surfaces 62a, 62b defined on second retainer lock shoulder portions 64a, 64b of the platform 32 are spaced from the tab rest surfaces 60a, 60b and define elongated slots 66a, 66b extending in the longitudinal direction parallel to the elongated channel 58, i.e., parallel to the longitudinal axis L₂. Hence, each slot 66a, 66b defines a continuous, straight line passage extending from an entrance end to an inner end of the slot 66a, 66b.

A second interface surface 68 extends generally perpendicular to the longitudinal axis L₂, wherein the interface surface 68 is defined at an interface between the first and second portions 58a, 58b of the channel 58, and the slots 66a, 66b can extend along the first channel portion 58a from the first side 46 of the platform 32 up to the longitudinal location of the interface surface 68. Hence, the slots 66a, 66b in the illustrated embodiment can generally extend the full length of the first portion 58a of the elongated channel 58, and the second interface surface 68 can be longitudinally displaced closer than the first interface surface 56 to the first side 46 of the platform 32.

The first portion 58a of the channel 58 may be formed as a generally semicircular surface, or as a section of a circle, having opposing edges at the tab rest surfaces 60a, 60b. The first portion 58a is located for engaging the retainer body 40a of the outlet tube retainer 40, and the tabs 40t extend radially from the retainer body 40a to laterally overlap the tab rest surfaces 60a, 60b and the tab retention surfaces 62a, 62b. Further, a vertical dimension of the slots 66a, 66b, as defined by a spacing between the tab rest surfaces 60a, 60b and the tab retention surfaces 62a, 62b can be slightly greater than the thickness T₂ of the tabs 40t to permit the tabs 40t to move in guided movement through the slots 66a, 66b. It may be understood that a clearance can be provided between the tabs 40t and the slot surfaces to avoid frictional binding and permit free sliding movement of the tabs 40t through the slots 66a, 66b.

The second and third portions 58b, 58c of the elongated channel 58 can be formed as U-shaped surface. The width dimension W_{b2} of the second portion 58b may define a width sufficient to receive the second end 30b of the tube section 30 positioned over the second port 40c of the outlet tube retainer 40. The width dimension W_{b3} of the third portion 58c may define a width sufficient to receive a portion of the tube section 30 adjacent to the second end 30b of the tube section 30 positioned over the second port 40c of the outlet tube retainer 40, and can be dimensioned to engage opposing sides of the tube section 30, i.e., the width dimension W_{b3} can be the same as, or slightly greater than, the outer diameter of the tube section 30.

Referring to Fig. 5, the tubing set 12 can be mounted to the pump 10 by placing the fluid supply tube retainer 38 in engagement within the first elongated channel 42. Placing the fluid supply tube retainer 38 can comprise positioning the tabs 38t on the shelf portions of the tab rest surfaces 44a, 44b, wherein the cylindrical shape of the cylindrical body 38a located within the generally semicircular first portion 42a of the channel 42 can facilitate aligning the fluid supply tube retainer 38 in centered relationship relative to the longitudinal axis L₁. An initial alignment of the fluid supply tube retainer 38, without the tube section 30 shown, can be seen in Fig. 4. The fluid supply tube retainer 38 may be moved forward toward the rotor 24 to engage the tabs 38t in sliding engagement within an entrance end of the slots 54a, 54b. The fluid supply tube retainer 38 may be further moved forward until it engages with the first interface surface 56 at a stop position, i.e., the tabs 38t engage against a stop position at an inner end of the slots 54a, 54b defined by the first interface surface 56 and/or a forward end of the cylindrical body 38a engages against the first interface surface 56. With the fluid supply tube retainer 38 engaged against the first interface surface 56, the second port 38c of the fluid supply tube retainer 38 and associated first end 30a of the tube section 30 is located within the second portion 42b of the channel 42, and opposing lateral sides of a portion of the tube section 30 are located adjacent to the third portion 42c of the channel 42.

The tube section 30 can be positioned around the outside of the rotor 24, extending from the inlet side 24a to the outlet side 24b. Subsequently, the tube section 30 can be elastically stretched by pulling the outlet tube retainer 40 to position the tabs 40t in front of the first side 46 of the platform 32, aligning the tabs 40t with an entrance end of respective slots 66a, 66b, as illustrated in Fig. 5. The outlet tube retainer 40 can be positioned forward to engage the tabs 40t in sliding engagement within the slots 66a, 66b. The outlet tube retainer 40 may be moved forward toward the rotor 24 until it engages with the second interface surface 68 at a stop position, i.e., the tabs 40t engage against a stop position at an inner end of the slots 66a, 66b defined by the second interface surface 68 and/or a forward end of the cylindrical body 40a engages against the second interface surface 68. With the outlet tube retainer 40 engaged against the second interface surface 68, the second port 40c of the outlet tube retainer 40 and associated second end 30b of the tube section 30 is located within the second portion 58b of the channel 58, and opposing lateral sides of a portion of the tube section 30 are located adjacent to the third portion 58c of the channel 58.

With the fluid supply tube retainer 38 engaged with the slots 54a, 54b and the outlet tube retainer 40 engaged with the slots 66a, 66b, the tube section 30 applies an elastic tension force that maintains the retainers 38, 40 in engagement with the respective interface surfaces 56, 68. That is, the elastic tension force of the tube section 30 resiliently biases the retainers 38, 40 to slide forward into engagement with the interface surfaces 56, 68 during operation of the pump 10. Further, the non-rotatable engagement of the tabs 3 8t, 40t with respective pairs of slots 54a, 54b and 66a, 66b can operate to resist twisting of the tube ends 30a, 30b during operation of the pump 10, i.e., twisting rotation of the tube ends 30a, 30b about the respective longitudinal axes L₁, L₂ can be resisted.

Referring further to Fig. 1, the housing 17 can be provided with a lid 70 that is pivotally mounted to the base 18. The lid 70 can have features that cooperate with corresponding elements of the retainers 38, 40. For example, an oval ridge 72 can be formed on an interior surface of the lid 70 for cooperating with an outer edge 74a of a vertically extending flange 74 on the fluid supply tube retainer 38, see Figs. 4 and 6, such as to limit longitudinal movement of the fluid supply tube retainer 38 when the lid 70 is closed. Additionally, the lid 70 can be formed with a retainer engaging rib 76 that can limit longitudinal movement of the outlet tube retainer 40 when the lid 70 is closed. Also, in the event that the tubing set 12 is installed incorrectly on the tubing set platform 32, such by reversing the location of the tube retainers 38, 40 relative to the retainer locks 34, 36, the oval ridge 72 and/or the retainer engaging rib 76 can engage against a respective misplaced tube retainer 40, 38, e.g., the rib 76 may engage against the flange 74, and prevent the lid 70 from fully closing. A small magnetic disc 84 may be provided in the lid 70 that can be sensed by a sensor (not shown) on the base 18, such that failure of the lid 70 to close properly can be sensed as an absence of the magnetic disc 84 being in a proximal sensed position, resulting in an alarm alerting a user to an error in the operating condition of the pump 10 and/or operation of the pump 10 may be disabled.

The lid 70 can further include tube positioning ribs 78a, 78b that can move into position over detection portions 30a₁, 30a₂ of the tube section 30, see Fig. 5, when the lid 70 is closed. The tube positioning ribs 78a, 78b can prevent the detection portions 30a₁, 30a₂ from bulging upward in the event that an occlusion in the tubing set 12 occurs during operation of the pump 10, wherein increased pressure caused by an occlusion can result in the detection portions 30a₁, 30a₂ being biased to expand sideways toward engagement with sides of the third portions 42c, 58c of the channels 42, 58. The portion of the tubing set platform 32 defining the third portions 42c, 58c of the channels 42, 58 can include ultrasonic sensors. In particular, the third portion 42c can be provided with an ultrasonic sensor including transmitter and receiver elements, generally depicted diagrammatically by 86a, 86b. Similarly, the third portion 58c can be provided with an ultrasonic sensor including transmitter and receiver elements, generally depicted diagrammatically by 88a, 88b.

It may be understood that causing or increasing a contact between the detection portions 30a₁, 30a₂ and the walls of the respective third portions 42c, 58c can change the transmittance of ultrasonic waves transmitted through the detection portions 30a₁, 30a₂, as generated and detected by the ultrasonic sensor transmitter and receiver elements 86a, 86b and 88a, 88b. Hence, a particular change in a detected ultrasonic signal can indicate the occurrence of an occlusion at either an upstream location, i.e., as detected by elements 86a, 86b, or at a downstream location, i.e., as detected by elements 88a, 88b.

Further referring to Fig. 2, the tubing set 12 includes a container or bag 80 that may contain a solution to be fed via the fluid supply tube 14, and can also include an anti-free flow connection 82, such as a check valve, at a distal end of the outlet tube 16. The anti-free flow connection 82 may comprise a disk valve that prevents fluid flow out of the outlet tube 16 at fluid pressures less than, for example, 13,79 - 34,47 kPa (2-5 psi).

The anti-free flow connection 82 prevents fluid from flowing through the tubing set 12 in the event that the tube section 30 becomes dislodged from engagement on the rotor 24. Locating the anti-free flow connection 82, including the check valve, at a distal end of the outlet tube 16 ensures that fluid is retained and present in the outlet tube 16 in the event that the tube 30 becomes dislodged and needs to be reinstalled, and thereby can avoid the need to prime the pump 10 before it is restarted.

Additionally, referring to Fig. 3, the second channel 58 may be provided with an infrared (IR) reflection detector, such as may be located within the tubing set platform 32 behind a window 90 in the second portion 58b of the second channel 58. IR light emitted from the detector through the window 90 can be reflected from the second end 30b of the tube section 30 and detected at the detector when the tube retainers 38 and 40 are positioned within the respective tube retainer locks 34, 36 to provide a verification that the tubing set 12 has been positioned within the pump 10.

## Claims

1. An enteral feeding pump (10) for mounting a tubing set (12), the enteral feeding pump (10) comprising:
a base (18) having an outer side (28);
a rotor (24) supported on the outer side (28) of the base (18) for engaging a tube section (30) of a tubing set (12);
a tubing set platform (32) on the outer side (28) of the base (18), the tubing set platform (32) defining a pair of tube retainer locks (34, 36), **characterized in that** at least one tube retainer lock (34, 36) comprises;
an elongated channel (42, 58) for receiving a tube retainer (38, 40) defined by a retainer body (38a, 40a) and a pair of laterally opposing retainer tabs (38t, 40t);
tab rest surfaces (44a, 44b, 60a, 60b) located on laterally opposing sides of the channel (42, 58);
tab retention surfaces (50a, 50b, 62a, 62b) spaced from the tab rest surfaces (44a, 44b, 60a, 60b) defining elongated slots (54a, 54b, 66a, 66b) extending in a longitudinal direction parallel to the elongated channel (42, 58);
wherein the elongated slots (54a, 54b, 66a, 66b) each include an entrance end for receiving a retainer tab (38t, 40t) and a longitudinally opposing inner end.

2. An enteral feeding pump (10) as set forth in claim 1, in combination with a tubing set (12):
the tubing set (12) comprising a tube section (30), first and second tube retainers (38, 40) connected to opposing ends of the tube section (30), each tube retainer (38, 40) comprising a retainer body (38a, 40a) and a pair of laterally opposing retainer tabs (38t, 40t); and wherein
the tubing set platform (32) of the enteral feeding pump (10) defines a pair of tube retainer locks (34, 36), each tube retainer lock (34, 36) comprising an elongated channel (42, 58) for receiving a retainer body (38a, 40a) of the tubing set (12).

3. The enteral feeding pump (10) as set forth in claim 1 or claim 2, wherein the elongated channel (42, 58) extends below a plane defined by the tab rest surfaces (44a, 44b, 60a, 60b).

4. The enteral feeding pump (10) as set forth in any preceding claim, wherein the elongated channel (42, 58) includes a first portion (42a, 58a) having a first lateral width (Wₐ₁) and a second portion (42b, 58b) having a second lateral width (Wₐ₂) that is less than the first lateral width (Wₐ₁), the first portion (42a, 58a) extending from at least a longitudinal location aligned with the entrance ends of the slots (54a, 54b, 66a, 66b) to a longitudinal location aligned with the inner ends of the slots (54a, 54b, 66a, 66b).

5. The enteral feeding pump (10) as set forth in claim 4, wherein an interface surface (56, 68), generally perpendicular to the longitudinal direction, is defined at an interface between the first and second portions (42a, 58a, 42b, 58b) for engaging an end of a retainer body (38a, 40a).

6. The enteral feeding pump (10) as set forth in claim 4 or claim 5, wherein the elongated channel (42, 58) further includes a third portion (42c, 58c) defining a tube passage having a third lateral width (Wₐ₃) that is less than the second lateral width (Wₐ₂).

7. The enteral feeding pump (10) as set forth in any preceding claim, wherein the elongated slots (54a, 54b, 66a, 66b) comprise passages for slidably receiving respective retainer tabs (38t, 40t).

8. The enteral feeding pump (10) as set forth in claim 7, wherein each of the passages comprise a continuous, straight line passage extending from the entrance end to the inner end of the slot (54a, 54b, 66a, 66b).

9. The enteral feeding pump (10) as set forth in any preceding claim, wherein at least one of the inner ends of the slots (54a, 54b, 66a, 66b) defines a stop surface for engaging and limiting sliding movement of the retainer tab (38t, 40t) within the slot (54a, 54b, 66a, 66b).

10. The enteral feeding pump (10) as set forth in any preceding claim, wherein each retainer tab (38t, 40t) includes a planar upper surface (38t₁, 40t₁) and a planar lower surface (38t₂, 40t₂) parallel to the planar upper surface (38t₁, 40t₁).

11. The enteral feeding pump (10) as set forth in any preceding claim, wherein each retainer body (38a, 40a) is a cylindrical body (38a, 40a) defining a fluid passage therethrough, and each channel (42, 58) is defined by a generally semicircular surface for engaging a respective retainer body (38a, 40a).

12. The enteral feeding pump (10) as set forth in any preceding claim, including an inlet tube (14) attached to one of the tube retainers (38, 40) and an outlet tube (16) attached to the other of the tube retainers (38, 40), and an anti-free flow check valve located at a distal end of the outlet tube (16).

13. A tubing set (12) in combination with an enteral feeding pump (10) in accordance with any of the preceding claims, **characterized in that** the tubing set (12) comprises:
a tube segment (30) having opposing ends (30a, 30b);
a tube retainer (38, 40) attached to each of the tube ends (30a, 30b), each tube retainer (38, 40) comprising a retainer body (38a, 40a) defining a longitudinal axis (L₁, L₂) and a pair of retainer tabs (38t, 40t) extending laterally outward from the longitudinal axis (L₁, L₂) in opposing directions, each retainer tab (38t, 40t) having upper and lower surfaces (38t₁, 40t₁, 38t₂, 40t₂) for guiding the tab (38t, 40t) through a respective retainer slot (54a, 54b, 66a, 66b).

14. The tubing set (12) as set forth in claim 13, wherein the upper and lower surfaces (38t₁, 40t₁, 38t₂, 40t₂) comprise a planar upper surface (38t₁, 40t₁) and a planar lower surface (38t₂, 40t₂) parallel to the planar upper surface (38t₁, 40t₁).

15. The tubing set (12) as set forth in claim 13 or claim 14, including an inlet tube (14) attached to one of the tube retainers (38, 40) and an outlet tube (16) attached to the other of the tube retainers (38, 40), and an anti-free flow check valve located at a distal end of the outlet tube (16).

## Patentansprüche

1. Pumpe (10) für die enterale Ernährung zum Montieren eines Schlauchsets (12), wobei die Pumpe (10) für die enterale Ernährung Folgendes umfasst:
eine Basis (18) mit einer Außenseite (28);
einen Rotor (24), der an der Außenseite (28) der Basis (18) getragen wird, um in einen Schlauchabschnitt (30) eines Schlauchsets (12) einzugreifen;
eine Schlauchset-Plattform (32) auf der Außenseite (28) der Basis (18), wobei die Schlauchset-Plattform (32) ein Paar von Schlauchhalteverschlüssen (34, 36) definiert, **dadurch gekennzeichnet, dass** mindestens ein Schlauchhalteverschluss (34, 36) umfasst:
einen länglichen Kanal (42, 58) zur Aufnahme eines Schlauchhalters (38, 40), der durch einen Haltekörper (38a, 40a) und ein Paar seitlich gegenüberliegender Haltelaschen (38t, 40t) definiert ist;
Laschenauflageflächen (44a, 44b, 60a, 60b), die sich auf seitlich gegenüberliegenden Seiten des Kanals (42, 58) befinden;
Laschenrückhalteflächen (50a, 50b, 62a, 62b), die von den Laschenauflageflächen (44a, 44b, 60a, 60b) beabstandet sind und die längliche Schlitze (54a, 54b, 66a, 66b) definieren, welche sich in Längsrichtung parallel zu dem länglichen Kanal (42, 58) erstrecken;
wobei die länglichen Schlitze (54a, 54b, 66a, 66b) jeweils ein Eingangsende zur Aufnahme einer Haltelasche (38t, 40t) und ein in Längsrichtung gegenüberliegendes inneres Ende einschließen.

2. Pumpe (10) für die enterale Ernährung (10) nach Anspruch 1 in Kombination mit einem Schlauchset (12):
wobei das Schlauchset (12) einen Schlauchabschnitt (30), einen ersten und einen zweiten Schlauchhalter (38, 40), die mit gegenüberliegenden Enden des Schlauchabschnitts (30) verbunden sind, umfasst, wobei jeder Schlauchhalter (38, 40) einen Haltekörper (38a, 40a) und ein Paar seitlich gegenüberliegender Haltelaschen (38t, 40t) umfasst; und wobei
die Schlauchset-Plattform (32) der Pumpe (10) für die enterale Ernährung ein Paar von Schlauchhalteverschlüssen (34, 36) definiert, wobei jeder Schlauchhalteverschluss (34, 36) einen länglichen Kanal (42, 58) zur Aufnahme eines Haltekörpers (38a, 40a) des Schlauchsets (12) umfasst.

3. Pumpe (10) für die enterale Ernährung nach Anspruch 1 oder Anspruch 2, wobei sich der längliche Kanal (42, 58) unterhalb einer Ebene erstreckt, die durch die Laschenauflageflächen (44a, 44b, 60a, 60b) definiert ist.

4. Pumpe (10) für die enterale Ernährung nach einem der vorstehenden Ansprüche, wobei der längliche Kanal (42, 58) einen ersten Abschnitt (42a, 58a) mit einer ersten seitlichen Breite (Wₐ₁) und einen zweiten Abschnitt (42b, 58b) mit einer zweiten seitlichen Breite (Wₐ₂), die geringer als die erste seitliche Breite (Wₐ₁) ist, einschließt, wobei sich der erste Abschnitt (42a, 58a) von mindestens einer Längsstelle, die mit den Eingangsenden der Schlitze (54a, 54b, 66a, 66b) ausgerichtet ist, zu einer Längsstelle erstreckt, die mit den inneren Enden der Schlitze (54a, 54b, 66a, 66b) ausgerichtet ist.

5. Pumpe (109 für die enterale Ernährung nach Anspruch 4, wobei eine Grenzfläche (56, 68), die im Allgemeinen senkrecht zur Längsrichtung verläuft, an einer Grenzfläche zwischen dem ersten und dem zweiten Abschnitt (42a, 58a, 42b, 58b) definiert ist, um ein Ende eines Haltekörpers (38a, 40a) in Eingriff zu nehmen.

6. Pumpe (10) für die enterale Ernährung nach Anspruch 4 oder Anspruch 5, wobei der längliche Kanal (42, 58) ferner einen dritten Abschnitt (42c, 58c) einschließt, der einen Schlauchdurchgang mit einer dritten seitlichen Breite (Wₐ₃) definiert, die geringer als die zweite seitliche Breite (Wₐ₂) ist.

7. Pumpe (10) für die enterale Ernährung nach einem der vorstehenden Ansprüche, wobei die länglichen Schlitze (54a, 54b, 66a, 66b) Durchgänge zur gleitenden Aufnahme jeweiliger Haltelaschen (38t, 40t) umfassen.

8. Pumpe (10) für die enterale Ernährung (10) nach Anspruch 7, wobei jeder der Durchgänge einen kontinuierlichen, geradlinigen Durchgang umfasst, der sich von dem Eingangsende zu dem inneren Ende des Schlitzes (54a, 54b, 66a, 66b) erstreckt.

9. Pumpe (10) für die enterale Ernährung nach einem der vorstehenden Ansprüche, wobei mindestens eines der inneren Enden der Schlitze (54a, 54b, 66a, 66b) eine Anschlagfläche für den Eingriff und die Begrenzung der Gleitbewegung der Haltelasche (38t, 40t) innerhalb des Schlitzes (54a, 54b, 66a, 66b) definiert.

10. Pumpe (10) für die enterale Ernährung nach einem der vorstehenden Ansprüche, wobei jede Haltelasche (38t, 40t) eine ebene obere Oberfläche (38t₁, 40t₁) und eine ebene untere Oberfläche (38t₂, 40tₜ₂) parallel zu der ebenen oberen Oberfläche (38tₜ₁, 40t₁) einschließt.

11. Pumpe (10) für die enterale Ernährung nach einem der vorstehenden Ansprüche, wobei jeder Haltekörper (38a, 40a) ein zylindrischer Körper (38a, 40a) ist, der einen Fluiddurchgang dort hindurch definiert, und jeder Kanal (42, 58) durch eine im Allgemeinen halbkreisförmige Oberfläche zum Eingriff mit einem jeweiligen Haltekörper (38a, 40a) definiert ist.

12. Pumpe (10) für die enterale Ernährung nach einem der vorstehenden Ansprüche, einschließend einen Einlassschlauch (14), der an einem der Schlauchhalter (38, 40) befestigt ist, und einen Auslassschlauch (16), der an dem anderen der Schlauchhalter (38, 40) befestigt ist, und ein Rückschlagventil gegen ungehinderten Durchfluss, das an einem distalen Ende des Auslassschlauchs (16) angeordnet ist.

13. Schlauchset (12) in Kombination mit einer Pumpe (10) für die enterale Ernährung nach einem der vorstehenden Ansprüche, **dadurch gekennzeichnet, dass** das Schlauchset (12) umfasst:
ein Schlauchsegment (30) mit gegenüberliegenden Enden (30a, 30b);
einen Schlauchhalter (38, 40), der an jedem der Schlauchenden (30a, 30b) befestigt ist, wobei jeder Schlauchhalter (38, 40) einen Haltekörper (38a, 40a), der eine Längsachse (L₁, L₂) definiert, und ein Paar Haltelaschen (38t, 40t), die sich von der Längsachse (L₁, L₂) in entgegengesetzte Richtungen seitlich nach außen erstrecken, umfasst, wobei jede Haltelasche (38t, 40t) obere und untere Oberflächen (38t₁, 40t₁, 38t₂, 40t₂) zum Führen der Lasche (38t, 40t) durch einen entsprechenden Halteschlitz (54a, 54b, 66a, 66b) aufweist.

14. Schlauchset (12) nach Anspruch 13, wobei die obere und die untere Oberfläche (38t₁, 40t₁, 38t₂, 40t₂) eine ebene obere Oberfläche (38t₁, 40t₁) und eine ebene untere Oberfläche (38t₂, 40t₂) parallel zu der ebenen oberen Oberfläche (38t₁, 40t₁) umfassen.

15. Schlauchset (12) nach Anspruch 13 oder Anspruch 14, einschließend einen Einlassschlauch (14), der an einem der Schlauchhalter (38, 40) befestigt ist, und einen Auslassschlauch (16), der an dem anderen der Schlauchhalter (38, 40) befestigt ist, und ein Rückschlagventil gegen ungehinderten Durchfluss, das an einem distalen Ende des Auslassschlauchs (16) angeordnet ist.

## Revendications

1. Pompe d'alimentation entérale (10) pour le montage d'un ensemble de tubulure (12), la pompe d'alimentation entérale (10) comprenant :
une base (18) ayant un côté extérieur (28) ;
un rotor (24) supporté sur le côté extérieur (28) de la base (18) pour s'engager avec une section de tube (30) d'un ensemble de tubulure (12) ;
une plate-forme d'ensemble de tubulure (32) sur le côté extérieur (28) de la base (18), la plate-forme d'ensemble de tubulure (32) définissant une paire de verrous de retenue de tube (34, 36), **caractérisée en ce qu'**au moins un verrou de retenue de tube (34, 36) comprend ;
un canal allongé (42, 58) pour recevoir un dispositif de retenue de tube (38, 40) défini par un corps de retenue (38a, 40a) et une paire de languettes de retenue (38t, 40t) latéralement opposées ;
des surfaces de repos de languette (44a, 44b, 60a, 60b) situées sur des côtés latéralement opposés du canal (42, 58) ;
des surfaces de rétention de languette (50a, 50b, 62a, 62b) espacées des surfaces de repos de languette (44a, 44b, 60a, 60b) définissant des fentes allongées (54a, 54b, 66a, 66b) s'étendant dans une direction longitudinale parallèle au canal allongé (42, 58) ;
dans laquelle les fentes allongées (54a, 54b, 66a, 66b) incluent chacune une extrémité d'entrée pour recevoir une languette de retenue (38t, 40t) et une extrémité intérieure longitudinalement opposée.

2. Pompe d'alimentation entérale (10) selon la revendication 1, en combinaison avec un ensemble de tubulure (12) :
l'ensemble de tubulure (12) comprenant une section de tube (30), un premier et un deuxième dispositif de retenue de tube (38, 40) reliés à des extrémités opposées de la section de tube (30), chaque dispositif de retenue de tube (38, 40) comprenant un corps de retenue (38a, 40a) et une paire de languettes de retenue latéralement opposées (38t, 40t) ; et dans laquelle
la plate-forme d'ensemble de tubulure (32) de la pompe d'alimentation entérale (10) définissant une paire de verrous de retenue de tube (34, 36), chaque verrou de retenue de tube (34, 36) comprenant un canal allongé (42, 58) pour recevoir un corps de retenue (38a, 40a) de l'ensemble de tubulure (12).

3. Pompe d'alimentation entérale (10) selon la revendication 1 ou la revendication 2, dans laquelle le canal allongé (42, 58) s'étend sous un plan défini par les surfaces de repos de languette (44a, 44b, 60a, 60b).

4. Pompe d'alimentation entérale (10) selon l'une quelconque des revendications précédentes, dans laquelle le canal allongé (42, 58) inclut une première partie (42a, 58a) ayant une première largeur latérale (Wₐ₁) et une deuxième partie (42b, 58b) ayant une deuxième largeur latérale (Wₐ₂) qui est inférieure à la première largeur latérale (Wₐ₁), la première partie (42a, 58a) s'étendant au moins d'un emplacement longitudinal aligné avec les extrémités d'entrée des fentes (54a, 54b, 66a, 66b) à un emplacement longitudinal aligné avec les extrémités intérieures des fentes (54a, 54b, 66a, 66b).

5. Pompe d'alimentation entérale (10) selon la revendication 4, dans laquelle une surface d'interface (56, 68), généralement perpendiculaire à la direction longitudinale, est définie à une interface entre la première et la deuxième partie (42a, 58a, 42b, 58b) pour s'engager avec une extrémité d'un corps de retenue (38a, 40a).

6. Pompe d'alimentation entérale (10) selon la revendication 4 ou la revendication 5, dans laquelle le canal allongé (42, 58) inclut en outre une troisième partie (42c, 58c) définissant un passage de tube ayant une troisième largeur latérale (Wₐ₃) qui est inférieure à la deuxième largeur latérale (Wₐ₂).

7. Pompe d'alimentation entérale (10) selon l'une quelconque des revendications précédentes, dans laquelle les fentes allongées (54a, 54b, 66a, 66b) comprennent des passages pour recevoir de manière coulissante des languettes de retenue respectives (38t, 40t).

8. Pompe d'alimentation entérale (10) selon la revendication 7, dans laquelle chacun des passages comprend un passage continu, en ligne droite, s'étendant de l'extrémité d'entrée à l'extrémité intérieure de la fente (54a, 54b, 66a, 66b).

9. Pompe d'alimentation entérale (10) selon l'une quelconque des revendications précédentes, dans laquelle au moins une des extrémités intérieures des fentes (54a, 54b, 66a, 66b) définit une surface d'arrêt pour s'engager avec et limiter le mouvement de coulissement de la languette de retenue (38t, 40t) à l'intérieur de la fente (54a, 54b, 66a, 66b).

10. Pompe d'alimentation entérale (10) selon l'une quelconque des revendications précédentes, dans laquelle chaque languette de retenue (38t, 40t) inclut une surface supérieure plane (38ti, 40ti) et une surface inférieure plane (38tz, 40t₂) parallèle à la surface supérieure plane (38ti, 40ti).

11. Pompe d'alimentation entérale (10) selon l'une quelconque des revendications précédentes, dans laquelle chaque corps de retenue (38a, 40a) est un corps cylindrique (38a, 40a) définissant un passage de fluide à travers celui-ci, et chaque canal (42, 58) est défini par une surface généralement semi-circulaire pour s'engager avec un corps de retenue respectif (38a, 40a).

12. Pompe d'alimentation entérale (10) selon l'une quelconque des revendications précédentes, incluant un tube d'entrée (14) attaché à l'un des dispositifs de retenue de tube (38, 40) et un tube de sortie (16) attaché à l'autre des dispositifs de retenue de tube (38, 40), ainsi qu'un clapet anti-retour situé à une extrémité distale du tube de sortie (16).

13. Ensemble de tubulure (12) en combinaison avec une pompe d'alimentation entérale (10) selon l'une quelconque des revendications précédentes, **caractérisé en ce que** l'ensemble de tubulure (12) comprend :
un segment de tube (30) ayant des extrémités opposées (30a, 30b) ;
un dispositif de retenue de tube (38, 40) attaché à chacune des extrémités de tube (30a, 30b), chaque dispositif de retenue de tube (38, 40) comprenant un corps de retenue (38a, 40a) définissant un axe longitudinal (L₁, L₂) et une paire de languettes de retenue (38t, 40t) s'étendant latéralement vers l'extérieur à partir de l'axe longitudinal (L₁, L₂) dans des directions opposées, chaque languette de retenue (38t, 40t) ayant des surfaces supérieure et inférieure (38ti, 40ti, 38t₂, 40t₂) pour guider la languette (38t, 40t) à travers une fente de retenue respective (54a, 54b, 66a, 66b).

14. Ensemble de tubulure (12) selon la revendication 13, dans lequel les surfaces supérieure et inférieure (38ti, 40ti, 38t₂, 40t₂) comprennent une surface supérieure plane (38ti, 40ti) et une surface inférieure plane (38t₂, 40t₂) parallèle à la surface supérieure plane (38ti, 40ti).

15. Ensemble de tubulure (12) selon la revendication 13 ou la revendication 14, incluant un tube d'entrée (14) attaché à l'un des dispositifs de retenue de tube (38, 40) et un tube de sortie (16) attaché à l'autre des dispositifs de retenue de tube (38, 40), et un clapet anti-retour situé à une extrémité distale du tube de sortie (16).
